# EUROPÄISCHE PATENTANMELDUNG

(11) **EP 1 754 498 A1**
(43) Veröffentlichungstag der Anmeldung: **21.02.2007**
(21) Anmeldenummer: 05018048.8
(22) Anmeldetag: 19.08.2005
(51) Int. Cl.: A61M 5/315, A61M 5/145

(54) **Antriebsvorrichtung für eine Injektions- oder Infusionsvorrichtung**

(71) Anmelder: Roche Diagnostics GmbH, 68305 Mannheim (DE); F.HOFFMANN-LA ROCHE AG, 4070 Basel (CH)
(72) Erfinder: Haar, Hans-Peter Dr, 69168 Wiesloch (DE); Harttig, Herbert Dr., 67434 Neustadt (DE)
(74) Vertreter: Jany, Peter

(57) **Zusammenfassung**

Es wird eine Antriebsvorrichtung (1) zum Vorschieben eines Kolbens (2) in einer Ampulle (3) vorgeschlagen, die eine sehr platzsparende, insbesondere kurze Bauweise ermöglicht. Hierzu wird ein Vorschubelement (6) verwendet, das aus Einzelgliedern gebildet ist, die nacheinander durch einen Umlenkbereich (7) in die Ampulle (3) eingeführt werden.

## Beschreibung

Die Erfindung betrifft eine Antriebsvorrichtung zum Vorschieben eines Kolbens in einer ein injizierbares Produkt enthaltenden Ampulle, wobei beim Vorschieben des Kolbens in der Ampulle enthaltenes Produkt aus der Ampulle in einen Körper abgegeben wird. Derartige Antriebsvorrichtungen umfassen ein längliches Vorschubelement, das zum Vorschieben des Kolbens in seiner Längsrichtung verschiebbar ist und mit dem zum Vorschieben des Kolbens eine Vorschubkraft auf den Kolben ausgeübt werden kann, und einen Antrieb zum Vorschieben des Vorschubelements. Um eine besonders kompakte Bauweise zu erzielen, ist es im Stand der Technik bekannt, einen Umlenkbereich vorzusehen, in dem das Vorschubelement in einem gekrümmten Abschnitt umgelenkt wird.

Bei der Injektion oder Infusion von flüssigen Produkten in einen Körper, beispielsweise von Medikamenten, werden nach dem Stand der Technik sogenannte Spritzenpumpen eingesetzt. Die Injektion oder Infusion kann dabei kontinuierlich oder diskontinuierlich erfolgen, wobei zumeist eine geeignete Steuerung zum Einstellen der injizierten Menge, der Injektionsgeschwindigkeit oder der Injektionszeitpunkte vorgesehen ist, um den Antrieb der Injektions- bzw. Infusionsvorrichtung entsprechend zu steuern. Durch den Antrieb wird der Kolben einer Spritze mittels eines geeigneten Stempels kontinuierlich oder diskontinuierlich nach vorne geschoben und dabei das in der Ampulle enthaltene Produkt, beispielsweise ein Medikament, über eine Schlauchverbindung und eine Nadel- oder Injektionskanüle einem Patienten injiziert.

Injektions- oder Infusionsvorrichtungen, die einen Antrieb zum kontinuierlichen oder diskontinuierlichen Abgeben von Medikamenten umfassen, sind gegenüber der Verwendung von Einmalspritzen vorteilhaft, wenn eine transkutane Verabreichung flüssiger Medikamente häufig oder über längere Zeit erforderlich ist. So ist es beispielsweise bei Diabetes bekannt, entweder täglich einzelne oder mehrere Insulinabgaben mittels einer Spritze zu verabreichen oder eine Vorrichtung zu verwenden, die ein Insulinreservoir umfaßt und für eine gewisse Dauer über einen Zugang an den Patienten angelegt wird, um kontinuierlich oder diskontinuierlich in kleinen Dosen Insulin abzugeben.

Die Verwendung einer tragbaren Injektions- oder Infusionsvorrichtung, die für eine gewisse Zeitdauer über einen Zugang Medikament an einen Körper mittels einer Pumpe abgeben kann, bedeutet beispielsweise für Patienten mit Diabetes beim Verabreichen von Insulin eine wesentliche Erleichterung und Verbesserung beim Umgang mit ihrer Krankheit. Die Zahl der transkutanen Injektionen wird reduziert und somit die damit verbundenen Schmerzen, mühsamen und umständlichen Handhabungsschritte und Infektionsgefahren. Darüber hinaus ist das Abgeben zahlreicher kleiner Insulindosen möglich, wodurch eine gezieltere Therapie möglich ist und es nicht zu so starken Spitzen- und Talkonzentrationen des Medikaments im Körper kommt, wie bei einer in größeren zeitlichen Abständen erfolgenden Verabreichung von transkutanen Injektionen mittels Spritzen.

Derartige Injektions- oder Infusionsvorrichtungen müssen hohe Anforderungen hinsichtlich ihrer Präzision, Zuverlässigkeit und einfachen Handhabung erfüllen. Eine in der Praxis ebenfalls sehr bedeutsame Anforderung stellen eine möglichst geringe Größe, kompakte Form und geringes Gewicht dar, damit der Patient die Vorrichtung unauffällig in oder unter seiner Kleidung oder unmittelbar am Körper tragen und verbergen kann, so daß sie Personen in seiner Umgebung nicht auffällt.

Aus dem Dokument WO 01/72358 A1 ist eine Insulinpumpe in Form einer Spritzenpumpe bekannt, bei der ein Teleskopantrieb verwendet wird, um eine kurze Baulänge der Antriebsvorrichtung zu erzielen. Diese bekannte Ausführungsform erfordert jedoch einen hohen konstruktiven Aufwand für den Antrieb mit Motor und Getriebe sowie der dazugehörigen Steuerung. Darüber hinaus benötigt der Antrieb an Platz mehr als ein Drittel der nutzbaren Verschiebeweglänge, so daß dadurch die Gesamtlänge des Geräts wesentlich über die Länge einer Ampulle hinausgeht und eine relativ lange Gesamtbaulänge der Injektionsvorrichtung resultiert.

In dem Dokument DE 100 48 220 A1 wurde eine Injektionsvorrichtung vorgeschlagen, die eine kurze Gesamtbaulänge realisieren soll. Dabei wird eine flexible Welle verwendet, um den Druck eines Stempels um 180° umzulenken und auf den Kolben der Ampulle wirken zu lassen. Auch diese bekannte Vorrichtung benötigt jedoch relativ viel Platz und weist keine optimal kurze Baulänge auf, da die vorgeschlagene flexible Welle einen Umlenkradius benötigt, der ein Vielfaches des Durchmessers der flexiblen Welle beträgt. Bei kleineren Biegeradien ist nämlich eine Umlenkung der Kraft nicht oder nur mit sehr hohen Reibungsverlusten möglich. Die in dem Dokument vorgesehene Umlenkung der flexiblen Welle sowie die zum Führen der flexiblen Welle erforderlichen, sich in Längsrichtung der Welle erstreckenden Außenfedern und Umlenkschläuche vergrößern den Platzbedarf und insbesondere die Baulänge der Vorrichtung beträchtlich.

Nachteilig bei den aus dem Stand der Technik bekannten Antriebsvorrichtungen sind somit insgesamt ihre Größe, ihr Gewicht und die durch die aufwendige Antriebsmechanik bedingten hohen Herstellungskosten.

Ausgehend von diesem Stand der Technik besteht die Aufgabe der Erfindung darin, eine Antriebsvorrichtung für eine Injektions- oder Infusionsvorrichtung zu schaffen, die eine möglichst kompakte Bauweise aufweist.

Diese Aufgabe wird erfindungsgemäß durch eine Antriebsvorrichtung mit den Merkmalen des Anspruchs 1 gelöst. Bevorzugte Ausgestaltungen und Weiterbildungen der Erfindung ergeben sich aus den abhängigen Ansprüchen und der nachfolgenden Beschreibung mit zugehörigen Zeichnungen.

Eine erfindungsgemäße Antriebsvorrichtung zum Vorschieben eines Kolbens in einer ein injizierbares Produkt enthaltenden Ampulle, wobei beim Vorschieben des Kolbens in der Ampulle enthaltenes Produkt aus der Ampulle in einen Körper abgegeben wird, umfaßt also ein längliches Vorschubelement, das zum Vorschieben des Kolbens in seiner Längsrichtung verschiebbar ist und mit dem zum Vorschieben des Kolbens eine Vorschubkraft auf den Kolben ausgeübt werden kann, einen Antrieb zum Vorschieben des Vorschubelements, und einen Umlenkbereich, in dem das Vorschubelement in einem gekrümmten Abschnitt umgelenkt wird. Ferner weist die Antriebsvorrichtung die Besonderheit auf, daß das Vorschubelement aus einer Reihe aufeinanderfolgender, untereinander gleichartig ausgebildeter Einzelglieder gebildet ist, die derart gestaltet und untereinander verbunden sind, daß sie nacheinander dem Umlenkbereich zugeführt werden können und nacheinander aus dem Umlenkbereich austreten und dabei derart zusammenwirken, daß sie die von dem Vorschubelement auf den Kolben ausgeübte Vorschubkraft von dem Antrieb entlang des Vorschubelements zu dem Kolben übertragen.

Mittels einer erfindungsgemäß ausgebildeten Antriebsvorrichtung ist es möglich, das Vorschubelement mit einem engen Umlenkradius in dem gekrümmten Abschnitt umzulenken, so daß dadurch eine sehr kompakte Bauform und insbesondere eine sehr kurze Baulänge der gesamten Injektionsvorrichtung erzielt werden kann.

Nach einem zusätzlichen vorteilhaften Merkmal wird vorgeschlagen, daß die Einzelglieder in einer Richtung quer zur Verschieberichtung des Vorschubelements beweglich untereinander verbunden sind. Durch die Beweglichkeit der Einzelglieder quer zur Verschieberichtung wird das Umlenken in einem engen Umlenkradius erleichtert.

Ein anderes vorteilhaftes Merkmal kann darin bestehen, daß das Vorschubelement von dem Antrieb nicht um die Längsrichtung des Vorschubelementes gedreht, sondern nur in Längsrichtung verschoben wird. Ein derartiger Antrieb kann gegenüber einem Antrieb, der das Vorschubelement um seine Längsrichtung dreht, kostengünstiger und einen schnelleren Vorschub realisierend ausgebildet werden.

Nach einem bevorzugten Merkmal wird vorgeschlagen, daß das Vorschubelement in dem Umlenkbereich um 180° umgelenkt wird. Dadurch liegt das Vorschubelement außerhalb der Ampulle parallel zur Ampulle und es wird eine besonders kompakte Baugröße erzielt.

Ein vorteilhaftes Ausbildungsmerkmal kann darin bestehen, daß von dem Antrieb vorgeschobene Einzelglieder in die Ampulle eingeführt werden, bevorzugt bis zum Kolben der Ampulle. Dadurch schieben die Einzelglieder den Kolben der Ampulle vor, wenn das Vorschubelement angetrieben wird, und werden in der Ampulle geführt.

Nach einem weiteren vorteilhaften Merkmal kann vorgesehen sein, daß ein Teil der Einzelglieder parallel zur Längsachse der Ampulle neben der Ampulle in einer Führung angeordnet ist, insbesondere wenn das Vorschubelement in dem Umlenkbereich um 180° umgelenkt wird. Es sind auch vorteilhafte Ausführungsformen möglich, bei denen eine Umlenkung um ca. 90° bis ca. 190° erfolgt.

Der Antrieb erfolgt vorzugsweise motorisch, insbesondere elektromotorisch. Er kann zum kontinuierlichen oder diskontinuierlichen Vorschieben des Vorschubelements ausgebildet sein und eine entsprechende Steuerung aufweisen, mittels der beispielsweise Vorschubgeschwindigkeit, Vorschubhübe und Vorschubzeitpunkte steuerbar sind.

Die Erfindung wird nachfolgend anhand zweier in den Figuren dargestellter Ausführungsbeispiele näher erläutert. Die darin beschriebenen Besonderheiten können einzeln oder in Kombination miteinander eingesetzt werden, um bevorzugte Ausgestaltungen der Erfindung zu schaffen. Insbesondere können die in jeweils einem Ausführungsbeispiel beschriebenen erfindungsgemäßen Merkmale auch in dem anderen Ausführungsbeispiel vorteilhaft sein. Es zeigen:
- Fig. 1: eine erfindungsgemäße Antriebsvorrichtung mit einer Spiralfeder,
- Fig. 2: ein Detail zu Figur 1 und
- Fig. 3: eine erfindungsgemäße Antriebsvorrichtung mit Kettengliedern.

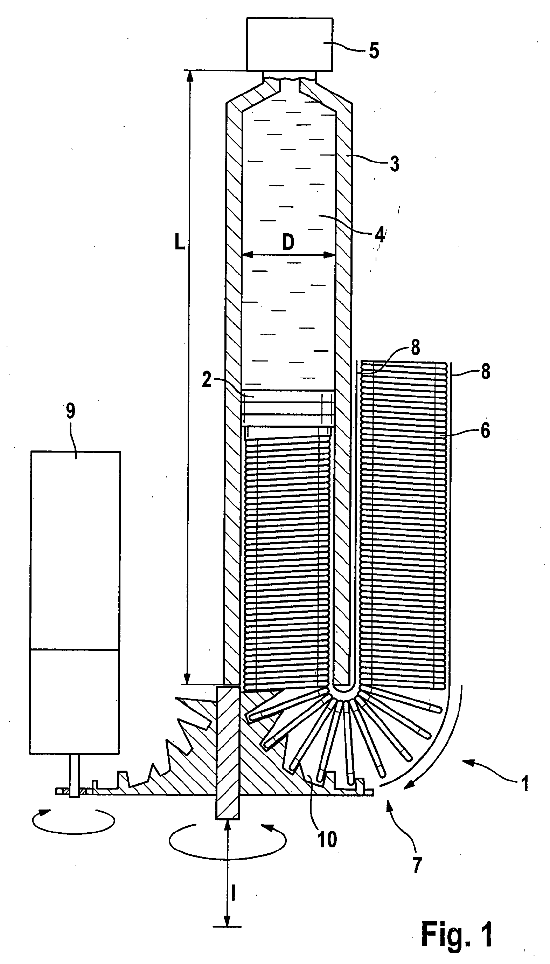

Die Figur 1 zeigt einen schematischen Querschnitt einer ersten Ausführungsform einer erfindungsgemäßen Antriebsvorrichtung 1 zum Verschieben eines Kolbens 2 in einer Ampulle 3. Die Ampulle 3 kann in beliebiger Weise gestaltet sein, beispielsweise aus Kunststoff oder aus Glas. Sie ist mit einem flüssigen Produkt 4, beispielsweise Insulin, gefüllt, das beim Vorschieben des Kolbens 2 an der Entnahmeöffnung 5 ausgedrückt wird. Die Ampulle 3 hat beispielsweise ein Volumen von 3 ml, eine Länge L von 65 mm und einen Innendurchmesser D von 9 mm.

Zum Vorschieben des Kolbens 2 in der Ampulle 3 dient ein längliches Vorschubelement, das in Figur 1 als Spiralfeder 6 ausgebildet ist. Das Vorschubelement in Form der Spiralfeder 6 umfaßt eine Reihe aufeinanderfolgender, untereinander gleichartig ausgebildeter Einzelglieder, nämlich in Figur 1 die Windungen der Spiralfeder 6, die derart gestaltet und untereinander verbunden sind, daß sie nacheinander einem Umlenkbereich 7 zugeführt werden können und nacheinander aus dem Umlenkbereich 7 austreten und dabei derart zusammenwirken, das sie die von dem Vorschubelement auf den Kolben 2 ausgeübte Vorschubkraft von dem Antrieb des Vorschubelements entlang des Vorschubelements zu dem Kolben 2 übertragen.

Dadurch, daß die Einzelglieder des Vorschubelements aus den Windungen einer Spiralfeder gebildet werden, sind die Einzelglieder in einer Richtung quer zur Verschieberichtung des Vorschubelements beweglich untereinander verbunden. Das erleichtert es, daß Vorschubelement in den Umlenkbereich 7 umzulenken. In Figur 1 erfolgt die Umlenkung des Vorschubelements in dem Umlenkbereich 7 um 180°. Dadurch kommt der noch nicht umgelenkte und in die Ampulle 3 eingeführte Teil der Spiralfeder 6 parallel zur Längsachse der Ampulle in einer entsprechenden Führung 8 zu liegen, wodurch eine sehr kompakte Bauform realisiert wird. Derjenige Teil der Spiralfeder 6, der umgelenkt ist, wird in die Ampulle 3 eingeführt und drückt beim Vorschieben auf den Kolben 2. Im Innenraum der Ampulle 3 reicht die Spiralfeder 6 bis zum Kolben 2.

Die von dem Antrieb vorgeschobenen Windungen der Spiralfeder 6 stabilisieren sich gegenseitig und bilden eine steife Röhre. Es ist daher in bevorzugten Ausführungsformen möglich, im Inneren, d.h. in Lumen, des von dem Antrieb vorgeschobenen Teils der Spiralfeder 6 kein die Bewegung der Spiralfeder 6 quer zu ihrer Verschieberichtung stabilisierendes Führungselement vorzusehen.

In der in Figur 1 dargestellten bevorzugten Ausführungsform liegen die von dem Antrieb vorgeschobenen Windungen der Spiralfeder 6 aufeinander, d.h. auf Block. Vorzugsweise ist die Spiralfeder 6 eine Zugfeder, bei der im unbelasteten Zustand alle Windungen aufeinander liegen. Die Stabilität der Spiralfeder 6 kann auch dadurch erhöht werden, daß ihre Windungen einen flachen Querschnitt aufweisen.

Die Spiralfeder 6 kann aus Metall oder Kunststoff gefertigt werden. Eine besonders hohe Steifigkeit des Materials ist dabei nicht notwendig. Die Fertigung der Spiralfeder 6 kann beispielsweise durch Wickeln eines Drahtes mit vorzugsweise flachem Querschnitt oder auch durch Schneiden eines Rohrabschnittes in Spiralform erfolgen.

Der Schnitt kann dabei in einem anderen Winkel als 90° gegen die Längsachse erfolgen.

Der Außendurchmesser der Spiralfeder 6 beträgt vorzugsweise zwischen 50% und 99%, bevorzugt zwischen 85% und 95% des Innendurchmessers D der Ampulle 3 oder des Innendurchmessers des Umlenkbereichs 7. Unter Innendurchmesser des Umlenkbereichs 7 ist dabei der Durchmesser der Führung oder der Öffnung, durch die die Spiralfeder 6 hindurchgeführt wird, zu verstehen. Dadurch ist gewährleistet, daß die Spiralfeder 6 in die Ampulle 3 eingeführt oder durch den Umlenkbereich 7 hindurchgeführt werden kann, ohne daß sie sich verklemmt, und dennoch eine ausreichende Stabilität zum Übertragen der Vorschubkraft aufweist. '

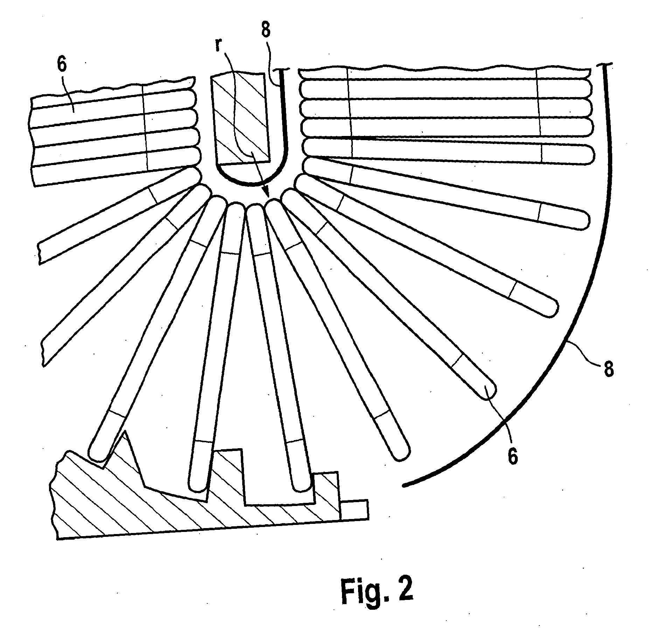

Aufgrund der erfindungsgemäßen Ausbildung des Vorschubelementes als Spiralfeder 6 kann die Spiralfeder 6 in dem Umlenkbereich 7 um einen Krümmungsradius r umgelenkt werden, der kleiner als der Außendurchmesser der Spiralfeder 6, bevorzugt kleiner als 50% des Außendurchmessers der Spiralfeder 6 und besonders bevorzugt kleiner als 30% des Außendurchmessers der Spiralfeder 6 ist. Bei dem in Figur 1 dargestellten Ausführungsbeispiel beträgt die dadurch erzielbare Verkürzung 1 der Baulänge mindestens 9 mm. In Figur 2 ist der Krümmungsradius r der Spiralfeder 6 veranschaulicht.

Der Antrieb zum Vorschieben der Spiralfeder 6 ist motorisch ausgebildet und umfaßt einen Getriebemotor 9 und ein Schneckenrad 10. Die Gänge des Schneckenrades 10 greifen in die einzelnen Windungen der Spiralfeder 6 ein und drücken sie in das Innere der Ampulle 3. Der Umlenkbereich 7 ist der Ampulle 3 vorgelagert, und der Antrieb steht in dem gekrümmten Abschnitt des Umlenkbereichs 7 mit der Spiralfeder 6 zum Erzeugen der Vorschubkraft in Eingriff, so daß die Spiralfeder 6 von dem Antrieb durch den gekrümmten Abschnitt geschoben und gezogen wird. Die Spiralfeder 6 wird also von dem Antrieb nicht um ihre Längsrichtung gedreht, sondern nur in Längsachse verschoben. Die dabei auftretenden Querkräfte können durch eine enge Führung in dem Umlenkbereich 7 oder in der Ampulle 3 aufgefangen werden. Zum Verringern von Reibung und Verschleiß können geeignete Materialpaarungen oder Beschichtungen ausgewählt werden.

Der Getriebemotor 9 oder ein anderes Element des Antriebs kann vorzugsweise auskuppelbar sein, um einen Wechsel der Ampulle 3 schnell durchführen zu können und um Antriebsenergie für das Zurückdrehen des Antriebs in die Ausgangslage einzusparen. Auf diese Weise können bei gegebener Batteriegröße längere Laufzeiten erzielt werden oder es kann eine kleinere Batterie verwendet werden.

An ihrem dem Kolben 2 zugewandten Ende kann die Spiralfeder 6 eine nicht dargestellte Kupplungsplatte tragen. Diese Kupplungsplatte kann Elemente umfassen, die eine kraftschlüssige Verbindung zum Kolben 2 der Ampulle 3 ermöglichen, beispielsweise eine Bajonett-Kupplung, ein Gewinde oder eine magnetische Kupplung.

Nach dem vollständigen Entleeren der Ampulle 3 wird diese von der Kupplungsplatte getrennt und das Schneckenrad 10 des Antriebs kann aus dem Eingriff in die Spiralfeder 6 bewegt werden. Danach wird die Spiralfeder 6 wieder in ihre Ausgangslage zurückgezogen und eine neue Ampulle 3 kann mit dem Antrieb verbunden werden.

Um eine ungewollte Bewegung des Kolbens 2 der Ampulle 3 und damit eine unbeabsichtigte Abgabe von Produkt 4 zu verhindern, kann der Kolben 2 mit einem nicht dargestellten Rückhalteelement verbunden sein. Eine ungewollte Bewegung des Kolbens 2 kann beispielsweise durch einen Unterdruck in dem Körper, in den die Injektion erfolgt, ausgelöst werden, oder durch Federkräfte der Spiralfeder 6, wenn deren Windungen zusammengepreßt werden. Ein solches Rückhalteelement kann beispielsweise in Form einer flexiblen Litze ausgebildet sein, die von dem Kolben 2 bzw. dem Ende der Spiralfeder 6 im Inneren der Spiralfeder 6 bis in die äußere Führung 8 für die Spiralfeder 6 geführt wird und dort an einer Zugfeder befestigt ist, die den Kolben 2 entgegen seiner Vorschubrichtung zieht oder spannt. Die Zugfeder kompensiert daher den möglichen statischen Unterdruck oder die vom Zusammenpressen der Windungen im Inneren der Ampulle 3 auf den Kolben 2 ausgeübten Vorschubkräfte, so daß sich der Kolben 2 nicht ungewollt, sondern nur beim Betätigen des Antriebs vorschiebt. Dadurch wird eine ungewollte Abgabe von Wirkstofflösung vermieden.

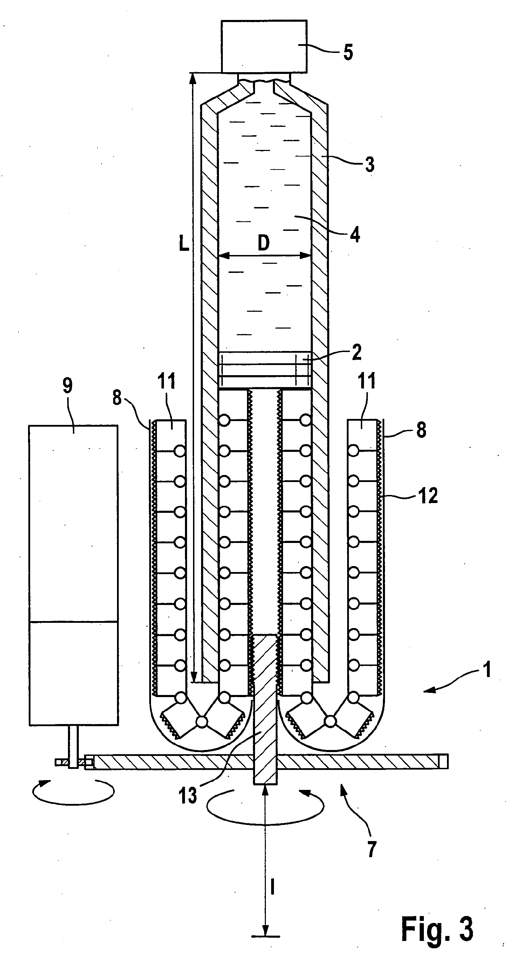

Die Figur 3 zeigt einen schematischen Querschnitt durch eine zweite erfindungsgemäße Ausführungsform. In dieser sind die Einzelglieder des Vorschubelements aus Kettengliedern 11 einer oder mehrerer Ketten gebildet. Die Kettenglieder können in Verschieberichtung der Kette, aber nicht quer dazu verschwenkt werden, und auf diese Weise die Vorschubkraft auf den Kolben 2 übertragen. Bevorzugt sind zwei, wie in Figur 3 dargestellt, oder drei Ketten vorgesehen.

Die Länge der Kettenglieder 11 ist vorzugsweise kleiner als der Innendurchmesser der Ampulle 3 oder der Innendurchmesser des Umlenkbereichs, bevorzugt kleiner als 50% und besonders bevorzugt kleiner als 30% des Innendurchmessers D, um ein leichtes Umlenken um einen kleinen Krümmungsradius zu ermöglichen. Die Kettenglieder 11 können aus Metall oder Kunststoff, bevorzugt im Druckguß- oder Spritzgußverfahren hergestellt sein.

Die gegenüber dem Stand der Technik erzielbare Verkürzung 1 beträgt bei dem in Figur 3 dargestellten Ausführungsbeispiel mindestens 12 mm.

Die Ampulle 3 weist die gleichen Abmessungen wie in Figur 1 auf. Die Kettenglieder 11 können nacheinander dem Umlenkbereich 7 zugeführt werden und aus dem Umlenkbereich 7 austreten und übertragen die Vorschubkraft auf den Kolben 2. Weitere Merkmale entsprechen denen der in Figur 1 dargestellten Ausführungsform, insbesondere folgende: Die Kettenglieder 11 sind in einer Richtung quer zur Verschieberichtung der Kette beweglich untereinander verbunden. Die Ketten werden in dem Umlenkbereich 7 um 180° umgelenkt. Die Ketten werden von dem Antrieb nicht um ihre Längsrichtung gedreht, sondern nur in Längsrichtung verschoben. Die von dem Antrieb vorgeschobenen Kettenglieder 11 werden in die Ampulle 3 eingeführt. Ein Teil der Kettenglieder 11 liegt parallel zur Längsachse neben der Ampulle 3 in einer Führung.

Auf ihrer Außenseite, d.h. der Seite, die bei der Umlenkung außen liegt, tragen die Kettenglieder 11 ein Gewinde 12. Diese Außenseiten der Kettenglieder 11 greifen mit ihrem Gewinde 12 in das Gewinde einer Gewindestange 13 ein, die von einem Getriebemotor 9 angetrieben wird. Dabei auftretende Querkräfte können durch eine enge Führung aufgefangen werden. Zum Verringern von Reibung und Verschleiß werden geeignete Materialpaarungen und/oder Beschichtungen gewählt.

Durch das Drehen der Gewindestange 13 werden die Kettenglieder 11 nach oben geschoben und gegen den Kolben 2 gedrückt. Der Antrieb greift also auf der Austrittseite des gekrümmten Abschnitts an den Ketten zum Erzeugen der Vorschubkraft an, so daß die Ketten von dem Antrieb durch den gekrümmten Abschnitt gezogen werden.

Die Kettenglieder 11 können an ihrem oberen Ende durch eine Kupplungsplatte miteinander verbunden werden. Diese Kupplungsplatte kann Elemente tragen, die eine kraftschlüssige Verbindung zum Kolben 2 ermöglichen, z.B. eine Bajonett-Kupplung, ein Gewinde oder eine magnetische Kupplung. Der Antrieb kann auch hier auskuppelbar sein, um eine schnelle Rückführung der Ketten in die Ausgangslage zu ermöglichen.

### Bezugszeichenliste

- 1: Antriebsvorrichtung
- 2: Kolben
- 3: Ampulle
- 4: Produkt
- 5: Entnahmeöffnung
- 6: Spiralfeder
- 7: Umlenkbereich
- 8: Führung
- 9: Getriebemotor
- 10: Schneckenrad
- 11: Kettenglieder
- 12: Gewinde
- 13: Gewindestange
- D: Innendurchmesser
- L: Länge
- 1: Verkürzung
- r: Krümmungsradius

## Patentansprüche

1. Antriebsvorrichtung (1) zum Vorschieben eines Kolbens (2) in einer ein injizierbares Produkt (4) enthaltenden Ampulle (3), wobei beim Vorschieben des Kolbens (2) in der Ampulle (3) enthaltenes Produkt (4) aus der Ampulle (2) in einen Körper abgegeben wird, umfassend
ein längliches Vorschubelement, das zum Vorschieben des Kolbens (2) in seiner Längsrichtung verschiebbar ist und mit dem zum Vorschieben des Kolbens (2) eine Vorschubkraft auf den Kolben (2) ausgeübt werden kann,
einen Antrieb zum Vorschieben des Vorschubelements, und
einen Umlenkbereich (7), in dem das Vorschubelement in einem gekrümmten Abschnitt umgelenkt wird,
**dadurch gekennzeichnet, daß**
das Vorschubelement aus einer Reihe aufeinanderfolgender, untereinander gleichartig ausgebildeter Einzelglieder gebildet ist, die derart gestaltet und untereinander verbunden sind, daß sie nacheinander dem Umlenkbereich (7) zugeführt werden können und nacheinander aus dem Umlenkbereich (7) austreten und dabei derart zusammenwirken, daß sie die von dem Vorschubelement auf den Kolben (2) ausgeübte Vorschubkraft von dem Antrieb entlang des Vorschubelements zu dem Kolben (2) übertragen.

2. Antriebsvorrichtung (1) nach Anspruch 1, **dadurch gekennzeichnet, daß** der Antrieb in dem gekrümmten Abschnitt mit dem Vorschubelement zum Erzeugen der Vorschubkraft in Eingriff steht, so daß das Vorschubelement von dem Antrieb durch den gekrümmten Abschnitt geschoben wird.

3. Antriebsvorrichtung (1) nach einem der vorhergehenden Ansprüche, **dadurch gekennzeichnet, daß** der Antrieb auf der Austrittsseite des gekrümmten Abschnitts an dem Vorschubelement zum Erzeugen der Vorschubkraft angreift, so daß das Vorschubelement von dem Antrieb durch den gekrümmten Abschnitt gezogen wird.

4. Antriebsvorrichtung (1) nach einem der vorhergehenden Ansprüche, **dadurch gekennzeichnet, daß** die Einzelglieder in einer Richtung quer zur Verschieberichtung des Vorschubelements beweglich untereinander verbunden sind.

5. Antriebsvorrichtung (1) nach einem der vorhergehenden Ansprüche, **dadurch gekennzeichnet, daß** das Vorschubelement in dem Umlenkbereich (7) um 90° bis 190°, bevorzugt um 180° umgelenkt wird.

6. Antriebsvorrichtung (1) nach einem der vorhergehenden Ansprüche, **dadurch gekennzeichnet, daß** das Vorschubelement von dem Antrieb nicht um die Längsrichtung des Vorschubelements gedreht, sondern nur in Längsrichtung verschoben wird.

7. Antriebsvorrichtung (1) nach einem der vorhergehenden Ansprüche, **dadurch gekennzeichnet, daß** von dem Antrieb vorgeschobene Einzelglieder in die Ampulle (3) eingeführt werden.

8. Antriebsvorrichtung (1) nach einem der vorhergehenden Ansprüche, **dadurch gekennzeichnet, daß** ein Teil der Einzelglieder parallel zur Längsachse der Ampulle (3) neben der Ampulle (3) in einer Führung (8) angeordnet ist.

9. Antriebsvorrichtung (1) nach einem der vorhergehenden Ansprüche, **dadurch gekennzeichnet, daß** die Einzelglieder des Vorschubelements aus Windungen einer Spiralfeder (6) gebildet sind.

10. Antriebsvorrichtung (1) nach Anspruch 9, **dadurch gekennzeichnet, daß** sich von dem Antrieb vorgeschobene Windungen der Spiralfeder (6) gegenseitig stabilisieren und eine steife Röhre bilden.

11. Antriebsvorrichtung (1) nach einem der Ansprüche 9 bis 10, **dadurch gekennzeichnet, daß** im Inneren des von dem Antrieb vorgeschobenen Teils der Spiralfeder (6) kein die Bewegung der Spiralfeder (6) quer zu ihrer Verschieberichtung stabilisierendes Führungselement angeordnet ist.

12. Antriebsvorrichtung (1) nach einem der Ansprüche 9 bis 11, **dadurch gekennzeichnet, daß** von dem Antrieb vorgeschobenen Windungen der Spiralfeder (6) aufeinander, d.h. auf Block liegen.

13. Antriebsvorrichtung (1) nach einem der Ansprüche 9 bis 12, **dadurch gekennzeichnet, daß** die Spiralfeder (6) eine Zugfeder ist.

14. Antriebsvorrichtung (1) nach einem der Ansprüche 9 bis 13, **dadurch gekennzeichnet, daß** die Windungen der Spiralfeder (6) einen flachen Querschnitt aufweisen.

15. Antriebsvorrichtung (1) nach einem der Ansprüche 9 bis 14, **dadurch gekennzeichnet, daß** der Außendurchmesser der Spiralfeder zwischen 50% und 99%, bevorzugt zwischen 85% und 95% des Innendurchmessers D der Ampulle (3) oder des Innendurchmessers des Umlenkbereichs (7) beträgt.

16. Antriebsvorrichtung (1) nach einem der Ansprüche 9 bis 15, **dadurch gekennzeichnet, daß** die Spiralfeder (6) in dem Umlenkbereich (7) um einen Krümmungsradius r umgelenkt wird, der kleiner als der Außendurchmesser der Spiralfeder (6), bevorzugt kleiner als 50% des Außendurchmessers der Spiralfeder (6) und besonders bevorzugt kleiner als 30% des Außendurchmessers der Spiralfeder (6) ist.

17. Antriebsvorrichtung (1) nach einem der Ansprüche 1 bis 8, **dadurch gekennzeichnet, daß** die Einzelglieder des Vorschubelements aus Kettengliedern (11) einer oder mehrerer Ketten gebildet sind.

18. Antriebsvorrichtung nach Anspruch 17, **dadurch gekennzeichnet, daß** die Länge der Kettenglieder (11) kleiner als der Innendurchmesser D der Ampulle (3) oder der Innendurchmesser des Umlenkbereichs (7) ist, bevorzugt kleiner als 50% und besonders bevorzugt kleiner als 30% des Innendurchmessers.
